# EUROPEAN PATENT APPLICATION

(11) **EP 4 082 414 A1**
(43) Date of publication of application: **02.11.2022**
(21) Application number: 21171160.1
(22) Date of filing: 29.04.2021
(51) Int. Cl.: A47K 5/12, A47K 5/14

(54) **A WEARABLE DISPENSER FOR CONTACTLESS HAND DESINFECTION**

(71) Applicant: ibf20 GmbH, 7000 Chur (CH); Bischof, Rino, 7000 Chur (CH)
(72) Inventor: Bischof, Rino, 7000 Chur (CH); Henkel, Michael, 61276 Weilrod (DE)
(74) Representative: Meyer-Dulheuer MD Legal Patentanwälte PartG mbB

(57) **Abstract**

The present invention provides a dispenser for contactless disinfection, comprising: a base module and a top case, configured to house a disinfectant cartridge; an electronic controlling unit; the disinfectant cartridge configured for containing disinfectant; a dosing mechanism, connected with the controlling unit, configured for storing and administrating disinfectant; a pump, respectively connected with the controlling unit, the disinfectant cartridge and the dosing mechanism, the pump being configured for supplying disinfectant to the dosing mechanism; a nozzle, connected with the dosing mechanism and configured for administrating the disinfectant to the hand; a power supply, configured for supplying a sufficient energy level of the dispenser. Said dispenser comprises an attaching mechanism, connected with the base module and configured for attaching the dispenser to human body. Said dispenser further comprises one or more sensor and one or more microprocessor, each of the sensor is connected with the respective microprocessor, the one or more microprocessor respectively is connected with the controlling unit, the sensor is configured for detecting an object, its temperature and its proximity with respect to the dispenser, and outputting a set of first data to the respective microprocessor. The microprocessor is configured for analyzing the set of first data from the respective sensor, and outputting a set of second data to the controlling unit. The controlling unit is configured for determining whether the object detected by the sensor is a human hand, the hand is located within a predefined distance and does not move for a predefined period based on the set of second data. The controlling unit is configured for instructing the dosing mechanism to administrate the disinfectant to the hand via the nozzle, and instructing the pump to supply the disinfectant from the disinfectant cartridge to the dosing mechanism.

## Description

### BACKGROUND OF INVENTION

### Field of Invention

The present invention relates to a dosage dispenser for contactless hand disinfection, and more particularly, to a wearable or portable dosage dispenser for contactless hand disinfection.

### Description of the state of the art

Hygienic hand disinfection is recognized worldwide as the most effective single measure for interrupting infection chains.

The conventional dispenser for stationary contactless hand disinfection is provided at the entrance of a building or a patient room or office. Furthermore, devices with manual pump mechanisms are available, or only pump bottles are provided, which must be taken into the hands of the user.

If the arm lever is not operated correctly, a user can even become infected by the devices. This is especially true if the dispenser has no fill level control. The user operates the lever, which can already be infectious but will not receive disinfectant.

With the conventional dosage dispensers for contactless hand disinfection, the form of the disinfectant is normally liquid. Drops of liquid can drip down from the palm of the hand, or the spray mist can pass the hand if the distance is too great.

It is therefore not guaranteed that the required amount of disinfectant according to the norm EN1500 reaches the hand and is available for trituration.

In particular, if the disinfectant is in the form of large droplets, the disinfectant may be wasted if it is applied next to or not completely on the palm before trituration. If the disinfectant is in the form of a spray, the disinfectant could get between the fingers or outside the palm.

### Problem to be solved

This means that in both cases, the disinfectant could flow everywhere, and it leads to a noticeable wastage of disinfectant, which calls into question the necessary effectiveness.

Users would have to initiate two pump strokes with most devices to get the correct amount of disinfectant, but many do not know this and therefore use too small amounts for hand disinfection.

Users would have to use their elbows to operate the lever to initiate the pump stroke. However, many users use their hand, possibly the infected palm of their hand, to actuate it.

The conventional dispenser also uses a motion sensor/detector. However, it is not suitable for wearable or mobile use, as human movement can lead to the unintentional triggering of the administration of the disinfectant.

A portable dispenser for hand disinfection can generally only contain a limited amount of disinfectant, so it is still preferable to avoid any waste of disinfectant.

Filling levels are not recorded electronically. There is a risk that the dispenser is empty. In this case, the conventional dispenser even becomes a source of infection.

Permanently installed dispensers create spots, as all persons gather in a very confined place and represent an additional risk of contamination.

As described above, the conventional devices are only suitable for stationary use and are not available for wearable or mobile use.

### SUMMARY OF INVENTION

### Solution to the problem

Therefore, the subject of the present invention is to solve the described problems, which is particularly practical and useful in view of the present situation of the current pandemic.

This problem is solved by performing a wearable dosage dispenser for contactless disinfection, said wearable dosage dispenser comprising: a base module and a top case, configured to house a disinfectant cartridge; an electronic controlling unit; the disinfectant cartridge, configured for containing different types of disinfectant for disinfection; a dosing mechanism, connected with the electronic controlling unit, configured for storing and administrating a required amount of disinfectant; a pump, respectively connected with the electronic controlling unit, the disinfectant cartridge and the dosing mechanism, the pump being configured for supplying disinfectant from the disinfectant cartridge to the dosing mechanism; a nozzle, connected with the dosing mechanism and configured for administrating the disinfectant from the dosing mechanism to the hand; a power supply, configured for supplying a sufficient energy level of the dispenser. Said wearable dosage dispenser further comprises an attaching mechanism connected with the base module and configured for attaching the dispenser to the human body. Said wearable dosage dispenser further comprises one or more sensor and one or more microprocessor, each of the sensor is connected with the respective microprocessor, the one or more microprocessor respectively is connected with the electronic controlling unit, the sensor is configured for detecting an object, its temperature and its proximity with respect to the dispenser, and outputting a set of first data to the respective microprocessor. The microprocessor is configured for analyzing the set of first data from the respective sensor and outputting a set of second data to the electronic controlling unit. The electronic controlling unit is configured for determining whether the object detected by the sensor is a human hand; the hand is located within a predefined distance and does not move for a predefined period based on the set of second data. The electronic controlling unit is further configured for instructing the dosing mechanism to administrate the required amount of disinfectant to the hand via the nozzle and instructing the pump to supply the disinfectant from the disinfectant cartridge to the dosing mechanism.

Alternatively, the nozzle is a foam nozzle, and the hand disinfectant is of foam property which prevents liquid from dripping down or spray mist from passing the palm of the human hand, thereby ensuring that a suitable amount of the disinfectant reaches the human hand.

Alternatively, the number of the sensor is one, and the number of the microprocessor is one.

Alternatively, the number of the sensor is two, the number of the microprocessor is one, both the first sensor and the second sensor are connected with the microprocessor, and the set of first data comprises a first data from the first sensor and a first data from the second sensor.

Alternatively, the number of the sensor is two, the number of the microprocessor is two, the first sensor is connected with the first microprocessor, and the second sensor is connected with the second microprocessor, and the set of first data comprises a first data from the first sensor and a first data from the second sensor, and the set of second data comprises a second data from the first microprocessor and second data from the second microprocessor.

Alternatively, the required amount of disinfectant is 0.5 ml to 3 ml.

Alternatively, the cartridge is replaceable, sealed, and refillable.

Alternatively, the battery is rechargeable, USB-C or via wireless energy transfer induction, or via a charging station.

Alternatively, the dispenser further comprises a slave RFID chip and a master RFID chip, and the slave RFID chip is mounted in the cartridge and configured for containing a set of third data about the disinfectant. The master RFID chip is wirelessly connected with the slave RFID chip and configured for receiving the set of third data from the slave RFID chip, the master RFID chip is connected with the controlling unit and configured for sending the set of third data to the controlling unit, and the controlling unit is further configured for determining to accept or reject the cartridge based on the set of third data.

Alternatively, the dispenser further comprises a docking part consisting of a coupling mechanism with a plug and a socket.

The present disclosure provides a method for administrating disinfectant via a wearable dispenser for contactless disinfection, said dispenser comprising one or more sensor, one or more microprocessor, an electronic controlling unit, a dosing mechanism, a pump, a nozzle, and a disinfectant cartridge. Said method comprises of the following steps: detecting an object, its temperature and its proximity with respect to the dispenser, and outputting a set of first data to the respective microprocessor; analyzing the set of first data, and outputting a set of second data to the electronic controlling unit; determining whether the detected object is a human hand, the hand is located within a predefined distance and does not move for a predefined period based on the set of second data; administrating the disinfectant to the hand via the nozzle; and supplying the disinfectant to the dosing mechanism.

### Advantageous Effects of the present disclosure

Contrary to the classical versions with arm lever and pump, the non-contact portable disinfectant dispenser has an integrated sensor that is triggered by detecting the human skin and dispenses the correct amount of disinfectant.

In daily use, it is important to ensure that the object in front of the dispenser is actually a human hand and not another part of the human body. In particular, if the disinfectant is emitted into face and eyes, e.g., of a child curiously looking at the dispenser, there is a medical risk. Therefore, it is necessary to distinguish between the human hand and other objects (e.g., face or etc.).

Since no touch is necessary, our dispenser is more hygienic and especially suitable for areas where a disinfectant dispenser is otherwise used by a large number of people. This includes, for example, hospitals, municipal medical facilities, doctors' practices, outpatient care services, and government institutions such as schools, kindergartens, and nursing homes, as well as laundries and kitchens used for providing direct supplies to patients. However, shopping markets, stores, and other businesses open to the public, as well as jobs in the non-domestic sector in general, are also affected.

### BRIEF DESCRIPTION OF DRAWINGS

Many aspects of the designs can be better understood with references to the following drawings. The components in the drawings are not necessarily true to scale, but the emphasis is on clearly illustrating the principles of the embodiments. In addition, identical reference numbers in the drawings identify the corresponding parts in two different drawings.

The invention itself can best be understood by means of the following detailed description of the invention, in which an exemplary embodiment of the invention is described in connection with the attached drawings, in which
Fig. 1 is a schematic representation of a first version of the top case according to the present invention.
Fig. 2 is a schematic representation of the disinfectant cartridge, which illustrates the embodiment according to the present invention.
Fig. 3 is a schematic representation of an embodiment of the basic module according to the present invention.
Fig. 4 is a schematic representation in which Figs. 1-3 of the present invention are shown in context.
Fig. 5 is a flow chart depicting the basic operation and signal flow of the dispenser according to the present invention.
Fig. 6 is a flow chart depicting the power-on sequence according to the present invention.
Fig. 7 is a schematic representation of connections between various components of the disinfectant cartridge according to a first embodiment of the present invention.

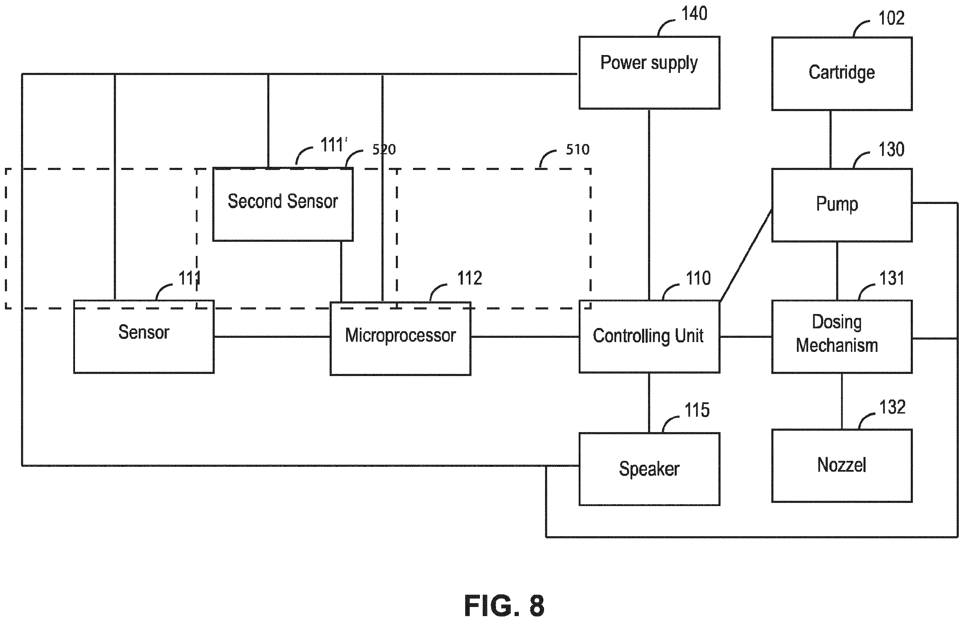

Fig. 8 is a schematic representation of connections between various components of the disinfectant cartridge according to a second embodiment of the present invention.

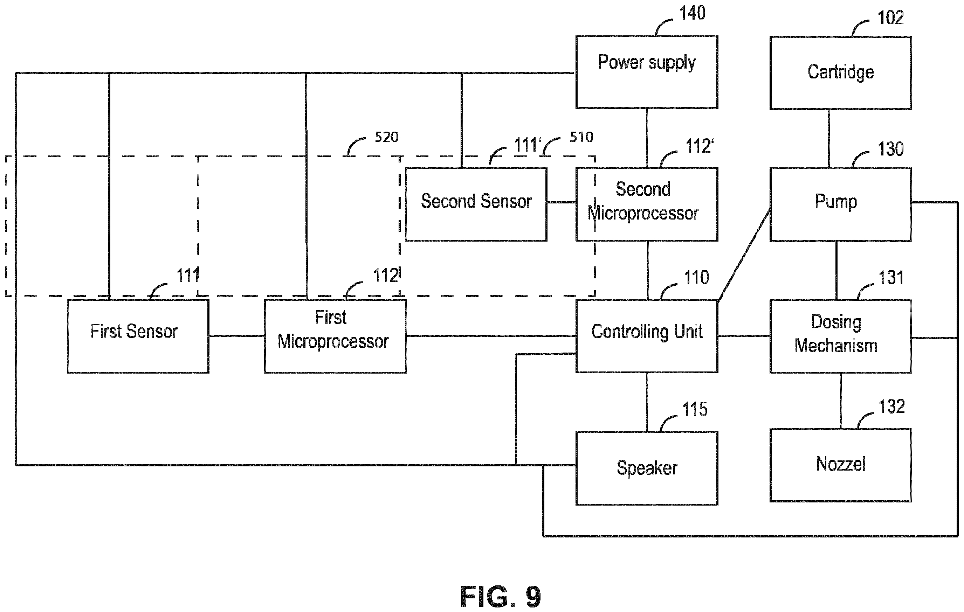

Fig. 9 is a schematic representation of connections between various components of the disinfectant cartridge according to a third embodiment of the present invention.

### DETAILED DESCRIPTION OF THE INVENTION

The present disclosure is described in more detail below with reference to the drawings and specific embodiments in order to understand better the objective, the technical solution, and the advantage of the present disclosure. It should be understood that the specific embodiments described here are merely illustrative and are not intended to limit the scope of the disclosure.

It should also be understood that the specific terms in this disclosure are interchangeable terms, e.g., "disinfectant", "fluid", or "foam" may be used interchangeably for "disinfectant fluid "; " electronic determining unit" or "electronic device" may also be used interchangeably.

In order to describe the present invention in detail, reference is now made to the drawing figures.

### BASIC STRUCTURE OF THE DISPENSER

The present dispenser is designed for individual use; therefore, no unnecessary agglomeration of people is created. The dispenser detects the human hand at the right distance and at rest and only then triggers a pump impulse. The present mechanism binds the disinfectant with an adjustable flow rate and dosing volume, which easily adheres to the palm of the hand and can be distributed correctly according to EN1500. The individual use of the dispenser ensures the highest possible compliance with EN1500 when used.

An audible signal informs the user about the dosage time until the end of the application. An audible signal also warns the user when the level of disinfectant is low.

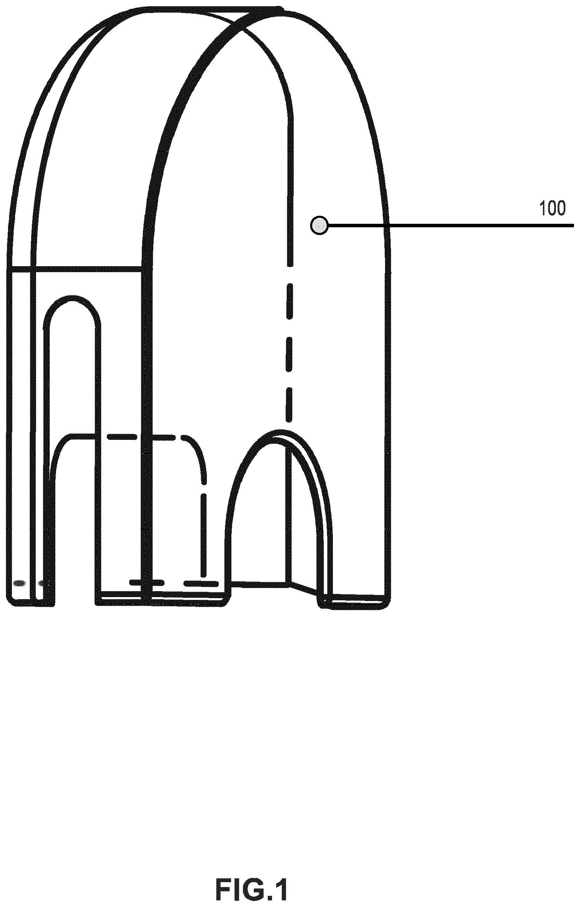

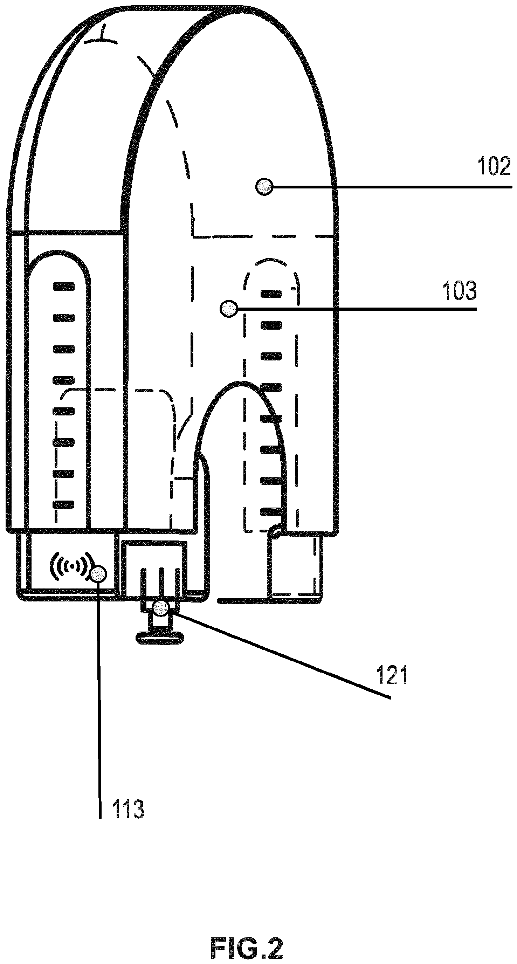

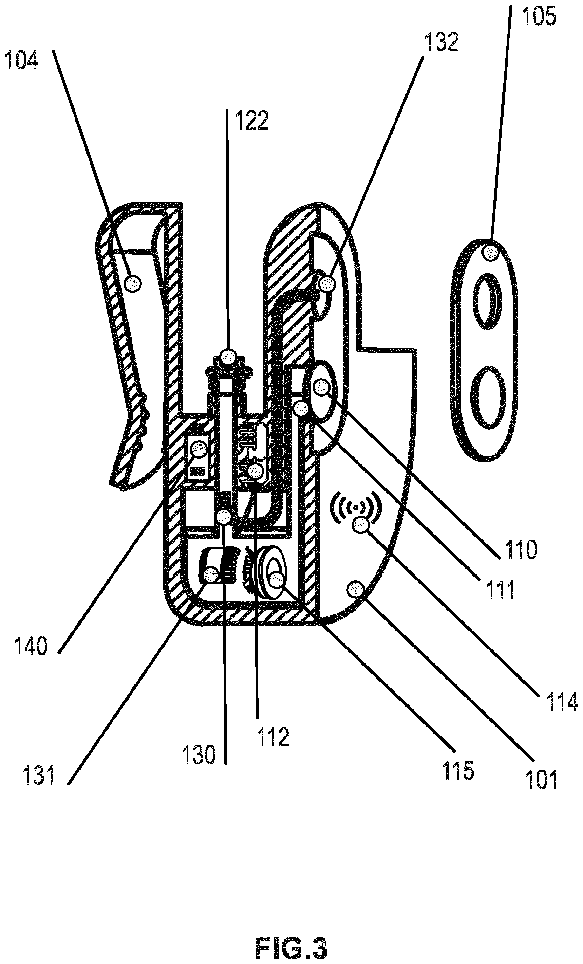

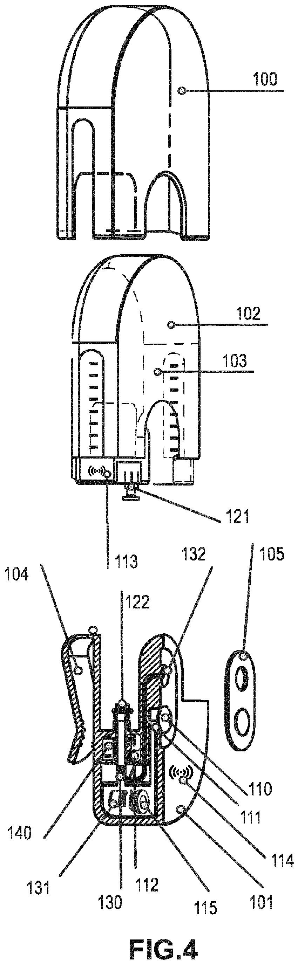

Referring to the **Figs. 1 to 4****,** the portable dosing dispenser for contactless hand disinfection comprises a base module 101 and a top case 100 configured to hold a disinfectant cartridge 102.

The disinfectant cartridge 102 is configured for containing different types of disinfectant for disinfection. The cartridge is replaceable, sealed, and refillable.

The portable dispenser comprises an electronic controlling unit 110 and a dosing mechanism 131 connected with the electronic controlling unit 110 for storing and administrating a required amount of disinfectant.

The portable dispenser comprises a pump 130 connected with the electronic controlling unit 110, the disinfectant cartridge 102, and the dosing mechanism 131, respectively. The pump 130 is configured for supplying disinfectant from the disinfectant cartridge 102 to the dosing mechanism 131.

The portable dispenser comprises a nozzle 132, connected with the dosing mechanism 131 and configured for administrating the disinfectant from the dosing mechanism 131 to the hand.

The dispenser further comprises one or more sensors 111 and one or more microprocessors 112. Each of the sensors 111 is connected with the respective microprocessor 112; the microprocessor 112 is respectively connected with the electronic controlling unit 110. The sensor 111 is configured for detecting an object, its temperature, and its proximity with respect to the dispenser and outputting a set of first data to the respective microprocessor 112. The microprocessor 112 is configured for analyzing the set of first data from the respective sensor 111 and outputting a set of second data to the electronic controlling unit 110.

The electronic controlling unit 110 is configured for determining whether the object detected by the sensor 111 is a human hand; the hand is located within a predefined distance and does not move for a predefined period based on the set of second data. The electronic controlling unit 110 is further configured for instructing the dosing mechanism 131 to administrate the required amount of disinfectant to the hand via the nozzle and instructing the pump 130 to supply the disinfectant from the disinfectant cartridge 102 to the dosing mechanism 131.

The controlling unit 110 is connected to a speaker 115 to provide an audible signal during the predefined period of time.

The predefined distance could be 1 cm to 15 cm but is not limited to this. The predefined period could be, but is not limited to, 0.5 second to 5 seconds.

The portable dispenser comprises a power supply 140 for supplying a sufficient energy level of the dispenser. The power supply 140 (e.g., battery) is connected to the sensor 111 and microprocessor 112, and the electronic controlling unit 110 and the dosing mechanism 131 and the pump 130 and is configured to provide sufficient power to the dosing dispenser. Battery 140 may be capable of providing sufficient power level to the dispenser for at least one hundred disinfections. If necessary, the battery 140 can also be integrated differently in the disinfection cartridge 102.

The battery is rechargeable, USB-C or via wireless energy transfer induction, or via a charging station.

In addition, the portable dosing dispenser is connected to a fastening mechanism 104, which is connected with the base module 101 and configured for attaching the dispenser to the human body or clothing. The mounting mechanism 104 could be, but is not limited to, a clip or any other component suitable for attaching the dispenser.

The nozzle is a foam nozzle, and the hand disinfectant is of foam property which prevents liquid from dripping down or spray mist from passing palm of the human hand, thereby ensuring that a suitable amount of the disinfectant reaches the human hand.

Alternatively, the number of the sensor 111 is one, and the number of the microprocessor 112 is one.

Alternatively, the number of the sensor 111 is two, the number of the microprocessor 112 is one. Both the first sensor 111 and the second sensor 111' are connected with the microprocessor 112, and the set of first data comprises a first data from the first sensor 111 and a first data from the second sensor 111'.

Alternatively, the number of the sensor is two, the number of the microprocessor is two. The first sensor 111 is connected with the first microprocessor 112, and the second sensor 111' is connected with the second microprocessor 112'. The set of first data comprises a first data from the first sensor 111 and a first data from the second sensor 111'. The set of second data comprises a second data from the first microprocessor 112 and second data from the second microprocessor 112'.

The required amount of disinfectant is 0.5 ml to 3 ml.

The dispenser further comprises a slave RFID chip 113 and a master RFID chip 114. The slave RFID chip 113 is mounted in the cartridge 102 and configured for containing a set of third data about the disinfectant. The master RFID chip 114 is wirelessly connected with the slave RFID chip 113 and configured for receiving the set of third data from the slave RFID chip 113. The master RFID chip 114 is connected with the controlling unit 110 and configured for sending the set of third data to the controlling unit 110. wherein the controlling unit 110 is further configured for determining to accept or reject the cartridge 102 based on the set of third data.

The dispenser further comprises a docking part consisting of a coupling mechanism with a plug 121 and a socket 122.

### BASIC OPERATION OF THE PROCESS

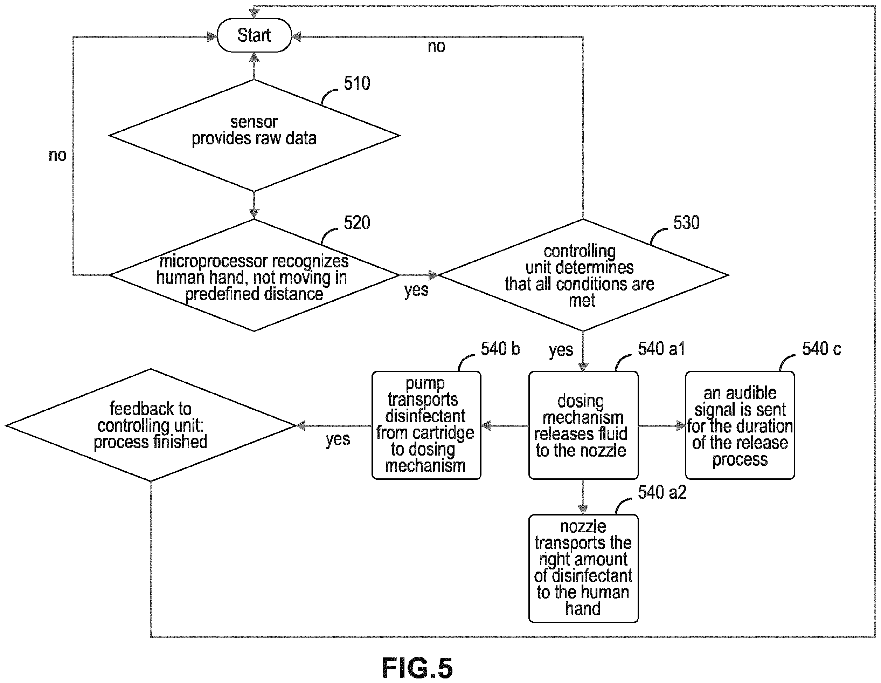

Referring to the **Fig. 5** of the portable dosing dispenser for contactless hand disinfection, the following embodiment describes the basic operation of the dispenser and how the elements of the embodiment interact, in order to achieve the desired result of dispensing an amount of disinfectant to the hand of the person using the dispenser.

When an object (for example, a human hand) is approaching near the dispenser (i.e., the sensors), the detecting mechanism detects that a human hand is held in front of the dispenser within the defined proximity.

The detecting mechanism comprises the one or more sensors 111 and the one or more microprocessors 112. The sensors 111 constantly collect raw data measurement and provides a set of first data to the microprocessors 112 to be analyzed.

The set of first data consist of data is obtained via the sensor's raw measurement. The raw measurement of the object via the detecting mechanism comprises, but not limited to, the temperature, the proximity or the distance with respect to the dispenser, the diameter and the shape.

### The first embodiment

In the first embodiment, the detecting mechanism comprises one sensor 111 and one microprocessor 112. The sensor 111 can be the first sensor 111, and the microprocessor 112 can be the first micro-processor 112.

**In Step 510,** when an object (e.g., human hand) approaches the dispenser and is held in front of the sensor 111, the infrared light from the sensor 111 is reflected by the object and correspondingly detected by a photoresistor of the sensor 111. The luminosity of the reflected light is evaluated by the sensor 111 and then output as a digital voltage signal from the sensor 111.

In the same way, the temperature of the object is detected and output as a digital voltage signal from the sensor 111. The sensor 111 is able to provide the temperature measurement (including thermal images) by a measuring area which is equivalent to 25 - 30 cm in diameter in the predefined distance range of 1 - 15 cm.

The sensor 111 then provides the set of first data to the microprocessors 112 to be analyzed.

**In Step 520,** the microprocessor 112 analyzes the set of first data and outputs a set of second data to the controlling unit 110.

The determining criteria are presented in the following. If the temperature of the object is detected in the range of 35 - 40 °C (degree Celsius), the object is determined as a human part (e.g., hand). The predefined proximity or the distance of the object detected with respect to the dispenser is in the range of 1 - 15 cm. The proximity is defined as in the range of 1 - 15 cm, preferably the range of 5 - 10 cm, and more preferably the range of 6 - 8 cm. The diameter of the object detected is in the range of 25 - 30 cm, while the virtual center of the virtual circle is the projection of the sensor 111 on the plane of the object. The shape of the human part is detected as a human hand with a splayed thumb. The predefined period during which the object does not move (i.e., stable or steady) is set in the range of 0.5 - 5 seconds.

The microprocessor 112 receives sets of first data from the sensor 111 which are used to determine whether the hand is not moving. By comparing the multiple thermal images representing the shape of a human hand with a splayed thumb, the microprocessor 112 can recognize that it does not move for a predefined period.

If all conditions are met as yes, the microprocessor 112 output the set of second data which is a confirmation that the object is the human hand stable for the predefined period and located within the predefined proximity.

The microprocessor 112 can recognize that a) the object is a human hand or not; b) the object is not moving for the predefined period; and c) the object is in the predefined distance or proximity. Via a bus line consisted of three data lines, the microprocessor 112 sends the confirmation for the above three conditions to the controlling unit 110.

Hence, the detecting mechanism sends confirmation to the electronic controlling unit 110 that a human hand is held steady in front of the dispenser within the defined proximity.

The sensor 111 coupled with the programmable microprocessor 112 may be from different manufactures and therefore should be treated separately. Alternatively, the sensor may also be two or more sensors coupled with one or more microprocessor 112. This will be presented in other embodiments.

**In Step 530,** The electronic controlling unit 110 determines from the set of second data that there is a human hand in the predefined proximity. The electronic controlling unit 110 also determines whether the human hand does not move for a predefined period of time.

If yes, the electronic controlling unit 110 then sends a first instruction to the dosing mechanism 131, in order to administer a quantity of disinfectant or disinfectant fluid. The dosing mechanism 131 administer disinfectant or disinfectant fluid which is stored via the nozzle 132 to the hand.

The controlling unit 110 also sends a second instruction to the pump 130, so as to refill and provide new disinfectant from the pump 130 to the dosing mechanism 131.

Generally, the inventor's trials have shown that the amount (quantity) of disinfectant to be used depends on the disinfectant that will be used for the specific application in a specific country.

The inventor's trials have shown that generally the foam with its amount between 23 ml and 28 ml (inclusive) is needed dependent on the various hands and the distance. Particularly, 26 ml of foam is a sufficient amount. Hence, the size of the hand is not a key factor.

For an example, when salt-based disinfectant is used, 2 ml of disinfectant fluid will expand to 26 ml of foam (which is sufficient amount). For another example, an alcohol-based disinfectant has half the expansion, hence 4 ml of disinfectant fluid would be needed.

The instruction can contain the duration of the flow from the container to the nozzle, so as to determine how much amount of disinfectant will be applied. The duration of the flow will be determined by the density, or volumic mass, of the disinfectant used and the ratio of the volumic expansion when turned into foam. The duration of the flow is dependent on the pump as well, namely, it takes 1 - 2 seconds for 0.5 - 3 ml of liquid that will expand to 25 - 30 ml of foam.

In an embodiment, the required amount of disinfectant is fixed. That is, the required amount of disinfectant is not variable during administrating or re-filling.

**In step 540a1**, the dosing mechanism 131 dispenses the quantity of disinfectant at an adjustable flow rate to the nozzle 132, based on the first instruction from the electronic controlling unit 110.

**In step 540a2,** the nozzle 132 applies the determined amount of disinfectant to the hand.

**In step 540b,** the pump 130 transports the quantity of disinfectant from the cartridge 102 to the dosing mechanism 131, based on the second instruction from the electronic controlling unit 110.

**In step 540c,** an audible signal is triggered by the electronic controlling unit 110 for the duration of the release process, so as to avoid that the hand is removed prematurely.

Once the release process is completed, the electronic controlling unit 110 is ready to repeat the process form step 510.

### The second embodiment

There are several embodiments possible for realizing the detection mechanism.

In an additional to the first embodiment, the present second embodiment further includes a sensor. For an example, the number of the sensor 111 is two, the number of the microprocessor 112 is also one. Both the first sensor 111 and the second sensor 111' are connected with the one single microprocessor 112. Because of the two sensors, the set of first data comprises a first data from the first sensor 111 and a first data from the second sensor 111'.

In this case and merely as an example, the first sensor 111 can be an (infrared) spectro sensor for detecting both the object and the temperature of the object, and then output a first data regarding object. The second sensor 111' can be an ultrasonic sensor for detecting the proximity (distance) of the object, and then output a first data regarding distance.

The sensors 111 and 111' collect raw data, and the microprocessor 112 analyzes the raw data and will determine what is needed, i.e., a) the object is a human hand; b) the object is not moving for the predefined period; and c) the object is in the predefined distance or proximity.

The inventor's trials have shown that at least two different measurements are needed for determining a) the object is a human hand; and b) the object is in the predefined distance or proximity. That is, a) an infrared sensor is needed for determining the object is a human hand; and b) an ultrasonic sensor is needed for determining whether the human hand is in the predefined distance or proximity.

That is, the proximity of the object or human hand is detected by another separate sensor, because potentially one sensor cannot precisely measure both distance and light (or temperature).

The microprocessor 112 then outputs the set of second data which is a confirmation that the object is the human hand stable for the predefined period and within the predefined proximity. It can be understood that other components of the wearable dispenser for hand disinfection are the same as the corresponding components in the first embodiment, and are not repeated herein.

### The third embodiment

In an additional to the second embodiment, the present third embodiment further includes a microprocessor. For another example, the number of the sensor is two, the number of the microprocessor is also two.

Thus, the first sensor 111 is connected with the first microprocessor 112, and the second sensor 111' is connected with the second microprocessor 112'.

The set of first data comprises a first data from the first sensor 111 and a first data from the second sensor 111', and the set of second data comprises a second data from the first microprocessor 112 and the second data from the second microprocessor 112'.

In this case and merely as an example, the first microprocessor 112 analyzes the first data from the first sensor 111 and then output a second data regarding object, which is a first confirmation that the object is the human hand stable for the predefined period.

The second microprocessor 112' analyzes the first data from the second sensor 111', and then output a second data regarding distance, which is which is a second confirmation that the object (human hand) is located within the predefined proximity.

It can be understood that other components of the wearable dispenser for hand disinfection are the same as the corresponding components in the first and second embodiments, and are not repeated herein.

### The fourth embodiment

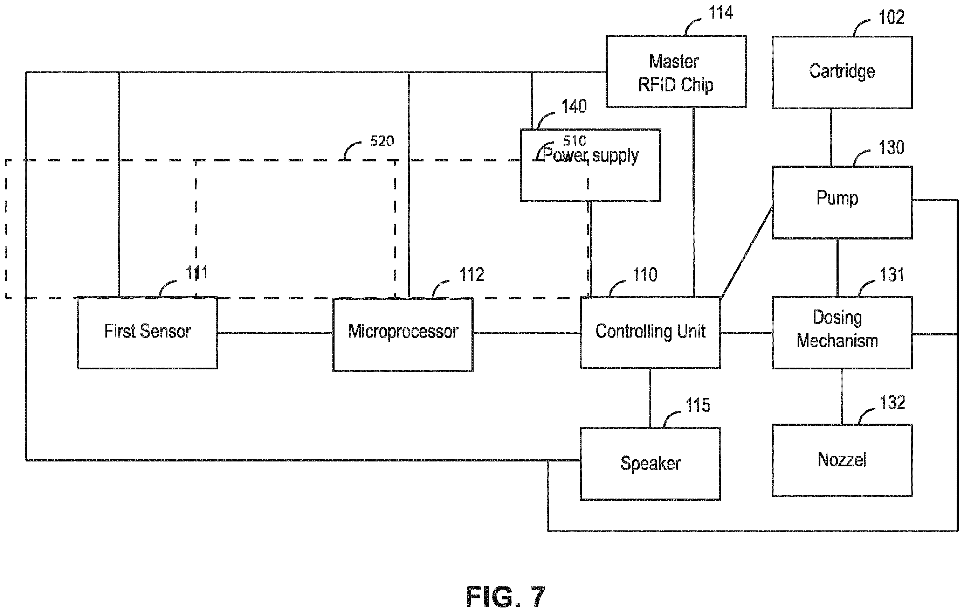

Referring to Fig. 7, the dispenser further comprises the master RFID chip 114 and the slave RFID chip 113.

The slave RFID chip 113 is mounted in the cartridge 102, and as an example, the slave RFID chip 113 is a passive tag or an adhesive chip. The slave RFID chip 113 is powered by energy from the master RFID chip 114 interrogating radio waves.

The slave RFID chip 113 contains information (i.e., being the set of third data) about the disinfectant contained in the cartridge 102. This set of third data can comprise, but is not limited to: a country specific accreditation (only use in Switzerland), an environment specific use (for healthcare, for food processing, etc.), the expiry date of the disinfectant, and the filling level at packaging. The information can be stored during filling of the cartridge 102.

The master RFID chip 114 is wirelessly connected with the slave RFID chip 113, and configured for receiving the set of third data from the slave RFID chip 113.

The master RFID chip 114 will be using the LF Band at 120 to 150 kHz; it generates radio waves to interrogate the slave RFID chip 113 and it also receives the authentication replies from the salve RFID chip 113. The authentication reply consists of a 96-bit string of data.

The slave RFID chip 113 modulates the field produced by the master RFID chip 114 by changing the electrical loading the slave RFID chip 113 represents. By switching between lower and higher relative loads, the slave RFID chip 113 produces a change that the master RFID chip 114 can detect.

The master RFID chip 114 is connected with the controlling unit 110, and sends the set of third data to the controlling unit 110.

Every time the dispenser is powered on, the master RFID chip 114 wirelessly communicates with the slave RFID chip 113, and sends the set of third data to the controlling unit 110.

The controlling unit 110 interprets and analyzes the set of third data, and determines to accept or reject (i.e., refuse) the cartridge 102.

Particularly, the disinfectant in the cartridge 102 needs to be certified with respect to specific uses, e.g., in certain applications (for healthcare, for food processing, etc.) or for certain countries (only use in Switzerland)
If the cartridge 102 is accepted, disinfectant will be supplied. Otherwise, if the cartridge 102 is rejected, no disinfectant will be supplied.

### The fifth embodiment

### Additional processes/operation

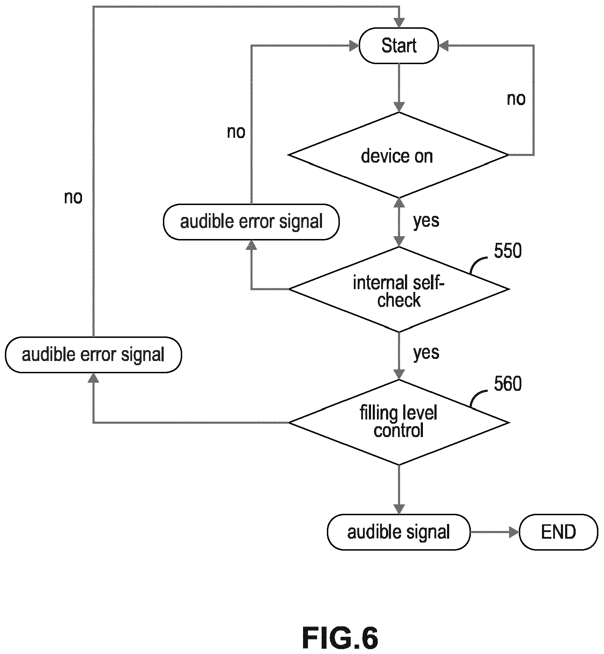

Referring to Fig. 6, the following embodiment describes the basic operation on power on. There are two additional relevant processes operations.

These processes are the same for all the above embodiments. It can be understood that the same steps as the corresponding steps in Fig. 5 are not repeated herein.

In Step 550, the electronic controlling unit 110 performs a self-test of the dispenser. If the self-test fails and audible error signal is triggered. If the self-test is OK, nothing further happens.

In Step 560, the electronic controlling unit 110 checks if the filling level of the cartridge is above a predetermined minimum. If the minimum is reached, an audible error signal is triggered. If the fill level is OK, nothing further happens.

When the dispenser is powered on, the two tests are conducted in sequence as illustrated in Fig. 6. Step 560 is periodically triggered at a predetermined interval, in order to avoid running out of disinfectant.

Although the disclosure has been described by way of example and in the sense of exemplary embodiment, it should be understood that disclosure is not limited to this. It is to be understood that the embodiment described above is merely illustrative and not restrictive. On the contrary, it is intended to design various modifications and similar embodiments for specific purposes (as would be apparent to those skilled in the art).

It will be easy for the those skilled in the art to understand that the above-mentioned form of execution can be supplemented, e.g. by the identification of the disinfectant approved for use with the help of RFID technology and or the logging of the use as well as the evaluation of the protocols for documentation purposes and that these can be superimposed without conflict. Various obvious or equivalent modifications or alterations of the details described above are included in the scope of the claims of this disclosure without deviating from the basic principles of the application. Therefore, the scope of the attached claims should be interpreted broadly enough to include all such modifications and similar arrangements.

## Claims

1. A wearable dosage dispenser for contactless disinfection, said wearable dosage dispenser comprising:
a base module (101) and a top case (100), configured to house a disinfectant cartridge (102); an electronic controlling unit (110);
the disinfectant cartridge (102), configured for containing different types of disinfectant for disinfection;
a dosing mechanism (131), connected with the electronic controlling unit (110), configured for storing and administrating a required amount of disinfectant;
a pump (130), respectively connected with the electronic controlling unit (110), the disinfectant cartridge (102) and the dosing mechanism (131), the pump (130) being configured for supplying disinfectant from the disinfectant cartridge (102) to the dosing mechanism (131);
a nozzle (132), connected with the dosing mechanism (131) and configured for administrating the disinfectant from the dosing mechanism (131) to the hand;
a power supply (140), configured for supplying a sufficient energy level of the dispenser;
**characterized in that** said wearable dosage dispenser further comprises:
an attaching mechanism (104), connected with the base module (101) and configured for attaching the dispenser to human body;
one or more sensor (111) and one or more microprocessor (112), each of the sensor (111) connected with the respective microprocessor (112),
the one or more microprocessor (112) respectively connected with the electronic controlling unit (110),
the sensor (111) being configured for detecting an object, its temperature and its proximity with respect to the dispenser, and outputting a set of first data to the respective microprocessor (112);
the microprocessor (112) being configured for analyzing the set of first data from the respective sensor (111), and outputting a set of second data to the electronic controlling unit (110),
wherein the electronic controlling unit (110) is configured for determining whether the object detected by the sensor (111) is a human hand, the hand is located within a predefined distance and does not move for a predefined period based on the set of second data; and
the electronic controlling unit (110) is further configured for instructing the dosing mechanism (131) to administrate the required amount of disinfectant to the hand via the nozzle, and instructing the pump (130) to supply the disinfectant from the disinfectant cartridge (102) to the dosing mechanism (131).

2. The wearable dispenser for hand disinfection in claim 1, wherein the nozzle is a foam nozzle, and the hand disinfectant is of foam property which prevents liquid from dripping down or spray mist from passing palm of the human hand, thereby ensuring that a suitable amount of the disinfectant reaches the human hand.

3. The wearable dispenser for hand disinfection in claim 1 or 2, wherein the number of the sensor (111) is one, and the number of the microprocessor (112) is one.

4. The wearable dispenser for hand disinfection in any one of the preceding claims, wherein the number of the sensor (111) is two, the number of the microprocessor (112) is one, both the first sensor (111) and the second sensor (111') are connected with the microprocessor (112), and wherein the set of first data comprises a first data from the first sensor (111) and a first data from the second sensor (111').

5. The wearable dispenser for hand disinfection in any one of the preceding claims, wherein the number of the sensor is two, the number of the microprocessor is two,
the first sensor (111) is connected with the first microprocessor (112), and the second sensor (111') is connected with the second microprocessor (112'), and
wherein the set of first data comprises a first data from the first sensor (111) and a first data from the second sensor (111'), and
the set of second data comprises a second data from the first microprocessor (112) and second data from the second microprocessor (112').

6. The wearable dispenser for hand disinfection in any one of the preceding claims, wherein the required amount of disinfectant is 0.5 ml to 3 ml.

7. The wearable dispenser for hand disinfection in any one of the preceding claims, wherein the cartridge is replaceable, sealed and refillable.

8. The wearable dispenser for hand disinfection in any one of the preceding claims, wherein the battery is rechargeable, USB-C or via wireless energy transfer induction, or via a charging station.

9. The wearable dispenser for hand disinfection in any one of the preceding claims, wherein the dispenser further comprises a slave RFID chip (113) and a master RFID chip (114), and wherein the slave RFID chip (113) is mounted in the cartridge (102) and configured for containing a set of third data about the disinfectant,
wherein the master RFID chip (114) is wirelessly connected with the slave RFID chip (113) and configured for receiving the set of third data from the slave RFID chip (113),
the master RFID chip (114) is connected with the controlling unit (110) and configured for sending the set of third data to the controlling unit (110), and
wherein the controlling unit (110) is further configured for determining to accept or reject the cartridge (102) based on the set of third data.

10. The wearable dispenser for hand disinfection in any one of the preceding claims, wherein the dispenser further comprises a docking part consisting of a coupling mechanism with a plug (121) and a socket (122).

11. A method for administrating disinfectant via a wearable dispenser for contactless disinfection, said dispenser comprising one or more sensor, one or more microprocessor, an electronic controlling unit, a dosing mechanism, a pump, a nozzle and a disinfectant cartridge,
said method comprising steps of:
detecting (510) an object, its temperature and its proximity with respect to the dispenser, and outputting a set of first data to the respective microprocessor;
analyzing (520) the set of first data, and outputting a set of second data to the electronic controlling unit;
determining (530) whether the detected object is a human hand, the hand is located within a predefined distance and does not move for a predefined period based on the set of second data;
administrating (540a) the disinfectant to the hand via the nozzle; and
supplying (540b) the disinfectant to the dosing mechanism (131).

12. The method in claim 11, wherein the nozzle is a foam nozzle, and the hand disinfectant is of foam property which prevents liquid from dripping down or spray mist from passing palm of the human hand, thereby ensuring that a suitable amount of the disinfectant reaches the human hand.
